# FASCICULE DE BREVET EUROPEEN

(11) **EP 0 768 299 B1**
(45) Date de publication et mention de la délivrance du brevet: **06.08.1997**
(21) Numéro de dépôt: 96401966.5
(22) Date de dépôt: 13.09.1996
(51) Int. Cl.: C07C 213/00, C07C 215/10

(54) **Procédé de préparation de 2-amino-alcane-1,3,4-triols**
Verfahren zur Herstellung von 2-Amino-1,3,4-Triol-Alkanen
Process for the preparation of 2-amino-1,3,4-triol-alkanes

(30) Priorité: 16.10.1995 FR 9512099
(43) Date de publication de la demande: 16.04.1997
(73) Titulaire: L'OREAL, 75008 Paris (FR)
(72) Inventeur: Semeria, Didier, 77181 Courtry (FR); Luppi, Bernadette, 93270 Sevran (FR); Philippe, Michel, 91230 Wissous (FR)
(74) Mandataire: Tezier Herman, Béatrice

(56) Documents cités:
- EP-A- 0 500 437
- EP-A- 0 646 572
- DE-C- 887 652
- HELVETICA CHIMICA ACTA, vol. XXXV, 15 Octobre 1952, Basel, CH, pages 1803-1805, XP000572379, M. VISCONTINI: "Réduction des substances bêta-amino-alpha, gamma-dicarbonylées; une nouvelle synthèse de l'allo-DL-phényl-3-amino-2-prppane-diol-1,3(DL-érythro-phénylsérinol", p. 1308-1805
- J. MARCH: "Advanced organic chemistry", 1985, John Wiley & Sons, XP002005742, page 1105
- Houben-Weyl, Methoden der Organischen Chemie, vol. XI/1, 1957, Georg Thieme Verlag, Stuttgart, DE, XP002005743, pages 495-506
- JOURNAL OF ORGANIC CHEMISTRY, vol. 34, no. 11, novembre 1969, Easton, US, pages 3539-3544, XP0000572350, K. SISIDO et al: "Synthesis of racemic phytosphingosine and the lyxo isomer"

## Description

L'invention a pour objet un nouveau procédé de préparation de précurseurs de céramides.

Les céramides, à l'état naturel, sont les composants principaux des couches lipidiques de l'épiderme. lls sont utilisés en cosmétique sous forme naturelle ou synthétique dans des compositions destinées, entre autre, à réduire le dessèchement de la peau ou à conférer à celle-ci une meilleure élasticité, ou encore destinées au traitement du cheveu.

Les céramides naturels sont généralement obtenus par extraction de la peau de porc, du cerveau de boeuf, de l'oeuf, des cellules du sang, des plantes (JP 86/260008 ou JP 86120308).

Les inconvénients nombreux liés à ce type d'approvisionnement (fragilité, contamination, conservation, coût, etc.) ont fait que très tôt la voie de la synthèse chimique a été explorée.

Les 2-amino-alcane-1,3,4-triols sont des intermédiaires connus de la synthèse de certains céramides, ce sont en particulier des précurseurs biologiques des céramides 3 et 6. De nombreuses voies de synthèse ont été développées pour la préparation de ces produits. En particulier des travaux ont été développés sur la synthèse des phytosphingosines (Prostenik, Chem. Phys. lipids 1971, 7, 135-143 ; Isida, J. Org. Chem. 1969, 34, 3539-3544 ; Jäger, Angew. Chem. Int. Ed. Engl. 1981, 20, 601-605), qui appartiennent à la famille des 2-amino-alcane-1,3,4-triols.

Prostenik propose une voie de synthèse en 10 étapes à partir de l'acide palmitique. Cette voie nécessite en particulier 3 niveaux de réduction différents et plusieurs étapes de protection et de déprotection, ce qui conduit à un rendement global très faible.

La voie proposée par Isida présente les mêmes inconvénients. En outre, le produit de départ qu'il emploie, l'acide pentadécanoïque, n'est pas commercial.

Enfin, Jäger décrit une préparation en trois étapes qui utilise des produits connus pour leur toxicité (hexaméthylphosphoramide, phénylisocyanate), dans des conditions non industrielles, comme une température de -78° C, avec une étape de purification délicate.

Cet ensemble de contraintes rend ces trois voies de synthèse inutilisables à l'échelle industrielle, dans une limite de coût acceptable, pour obtenir les 2-amino-alcane-1,3,4-triols, précurseurs de céramides.

La demanderesse a donc cherché à améliorer les voies de synthèse des céramides et en particulier de leurs précurseurs, les 2-amino-alcane-1,3,4-triols.

Après de longues recherches et de manière surprenante et inattendue, contrairement à ce que l'art antérieur enseigne de manière constante depuis de longues années, la demanderesse a découvert que la voie de synthèse directe à partir d'un alkyl-2-oximino-3-oxo-4-acyloxyalcanoate en une seule étape de réduction peut conduire à un 2-amino-alcane-1,3,4-triol.

Supprimant de nombreuses étapes, l'invention permet un gain de temps très substantiel et un rendement de synthèse très amélioré, qui industriellement diminuent le prix de revient des céramides.

Ainsi, l'invention a pour objet un procédé de préparation de 2-amino-alcane-1,3,4-triols caractérisé par une réduction en une seule étape des fonctions réductibles d'un alkyl-2-oximino-3-oxo-4-acyloxyalcanoate de formule (I) : 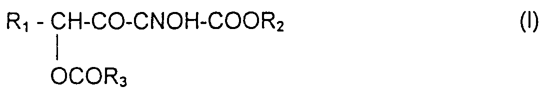 dans laquelle
R₁ représente un radical alcoyle, un radical alcényle ou un radical aralcoyle, éventuellement interrompu par des ponts éthers, linéaire ou ramifié, éventuellement hydroxylé et/ou porteur d'une fonction acyloxy en C₁ à C₈, comprenant 4 à 28 atomes de carbone,
R₂ représente un radical alcoyle ou un radical alcényle, éventuellement interrompu par des ponts éthers, linéaire ou ramifié, comprenant 1 à 5 atomes de carbone,
R₃ représente un radical alcoyle ou un radical alcényle, ou un radical aryle, linéaire ou ramifié, comprenant 1 à 6 atomes de carbone,
dans un solvant, en présence d'au moins un hydrure.

De manière avantageuse, R₁ représente un radical alcoyle ou un radical alcényle, éventuellement hydroxylé, ayant de 8 à 20 atomes de carbone.

Selon un mode préféré de réalisation de l'invention, R₂ est un radical méthyle ou éthyle.

De façon avantageuse, on choisit R₃ parmi les radicaux méthyle ou phényle.

Le procédé selon l'invention permet la synthèse en une étape des 2-amino-alcane-1,3,4-triols répondant à la formule (II):

R₁-CHOH-CHOH-CHNH₂-CH₂-OH (II)

ou des sels correspondants, dans laquelle R₁ a la même signification que ci-dessus.

Le composé de formule (II) se présente après synthèse sous la forme d'un mélange de stéréoisomères (énantiomères et/ou diastéréoisomères).

Le solvant est avantageusement anhydre. Par exemple, on peut citer comme solvant le toluène, l'heptane, le tétrahydrofurane, le tertiobutylméthyléther ou encore l'éther isopropylique. De manière préférentielle, on utilise le tertiobutylméthyléther.

De façon préférentielle, la réaction est réalisée sous atmosphère inerte, afin d'éviter la dégradation du réactif par de l'air ou de l'eau. Pour cela on utilise généralement l'azote ou l'argon. De manière préférentielle, on réalise la réaction sous atmosphère composée d'argon.
De façon préférentielle, la réaction est commencée à une température initiale contrôlée. Par température initiale contrôlée, on entend que la réaction de réduction s'initie à une température allant de -10° C à la température ambiante. De préférence, la réaction est initiée à 0°C.

La réaction peut se poursuivre à toute température comprise entre -10° C et la température de reflux du solvant utilisé. Avantageusement, la réaction est réalisée à la température de reflux du solvant utilisé.

Parmi les hydrures utilisables selon l'invention, on peut citer l'aluminohydrure de lithium (LiAlH₄) ou encore le bis-(2-méthoxy-éthoxy)-dihydroaluminate de sodium (Red-AI™ vendu par la société Aldrich ou vitride vendu par la Société HEXCEL). De manière préférentielle, on utilise le bis-(2-méthoxy-éthoxy)-dihydroaluminate de sodium.

En fin de réaction le produit synthétisé peut être sous forme de complexes d'aluminium. Avantageusement, pour limiter la formation de ces complexes, des quantités minimales d'hydrure sont utilisées.

Ainsi, l'hydrure est généralement présent dans le milieu réactionnel à une concentration allant de 3 à 7 équivalents-molaires par rapport à l'alkyl-2-oximino-3-oxo-4-acyloxy alcanoate. De préférence, on utilise une concentration allant de 3 à 6 équivalentsmolaires par rapport à l'alkyl-2-oximino-3-oxo-4-acyloxyalcanoate.

A la fin de la réaction, marquée par la disparition des produits de départ, pour détruire les complexes éventuellement formés, on amène préférentiellement le pH du milieu réactionnel à une valeur inférieure à 2 ou supérieure à 11, respectivement par addition au milieu réactionnel d'acide ou de base que l'on utilise sous la forme d'une solution aqueuse.

Préférentiellement, le pH du milieu réactionnel est voisin de 1 ou voisin de 12. Ainsi, de préférence on utilise de l'acide chlorhydrique ou de la soude respectivement.

Les alkyl-2-oximino-3-oxo-4-acyloxy alcanoates répondant à la formule (I) peuvent être aisément préparés par des moyens connus de l'homme du métier. Pour leur synthèse, on pourra par exemple se référer au document EP-A-0646572.
On pourra également utiliser comme produits de départ les alkyl-3-oxoalcanoates décrits dans la demande de brevet français déposée sous le numéro d'enregistrement national 95-12041 et répondant à la formule (III): 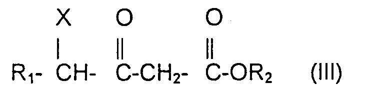 dans laquelle
R₁ et R₂ ont la même signification que précédement et X désigne un groupe partant, comme défini par Jerry March dans 〈〈 Advanced Organic Chemistry 〉〉, Wiley Interscience, 3è édition p315 table 10, et de façon préférentielle X désigne un atome de brome ou de chlore ou un groupement sulfonate.

On peut par exemple préparer les produits de formule (III) en traitant un dérivé malonique avec un agent acylant. Par dérivé malonique, on désigne un monoester ou un diester de l'acide malonique, comme par exemple l'ester d'isopropylidène de l'acide malonique appelé aussi acide de Meldrum ou le sel de potassium du malonate d'éthyle. La réaction est de préférence conduite en milieu anhydre. Elle est avantageusement réalisée dans un solvant approprié, comme par exemple le tétrahydrofurane, le dichlorométhane, la pyridine, le tertiobutylméthyl éther.

Par substitution nucléophile, les composés alkyl-3-oxoalcanoates répondant à la formule (III) conduisent facilement à des dérivés répondant à la formule (IV) 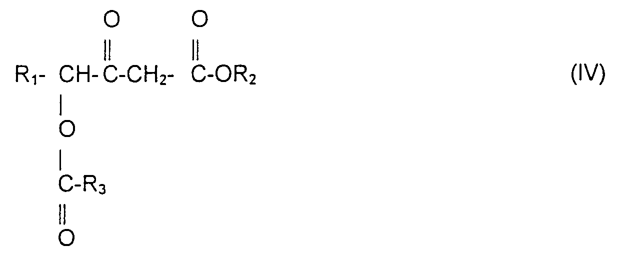 dans laquelle
R₁, R₂ et R₃ ont la même définition que précédement.

Les alkyl-3-oxo-4-acyloxyalcanoates selon la formule (IV) conduisent ensuite facilement aux alkyl-2-oximino-3-oxo-4-acyloxyalcanoates selon la formule (I). Par exemple, on peut les faire réagir avec un nitrite d'alkyle en milieu anhydre acidifié par de l'acide chlorhydrique gazeux. Par nitrite d'alkyle, on entend un composé ayant pour formule RONO, dans laquelle R représente un radical alkyle ayant de 2 à 6 carbones, comme par exemple le nitrite de butyle.

Le composé de formule (II) obtenu selon l'invention peut être utilisé dans les procédés de synthèse pouvant conduire à des céramides comme ceux décrits par Shapiro, D. (Chemistry of sphingolipids, Hermann, Paris, 1969, p 26-34).

Ainsi, par exemple il est possible de préparer le céramide recherché, par acylation de la fonction amine d'un 2-amino-alcane-1,3,4-triol par un chlorure d'acide, par un anhydride, par un ester de paranitrophénol, par un ester de succinimide, par un ester de dicyclohexylcarbodiimide, par un ester de méthyle ou d'éthyle ou par un azolide comme notamment un imidazolide ou un pyrazolide, en milieu anhydre ou dans un solvant tel que le tétrahydrofuranne, la pyridine, le diméthylformamide ou le dichlorométhane. Avantageusement, l'anhydride est un anhydride mixte et l'azolide est un imidazolide ou un pyrazolide.

On va donner maintenant, sans effet limitatif, des exemples de synthèse selon l'invention.

### Exemples:

### Exemple 1 : Synthèse du 2-amino-octadécane-1,3,4-triol

Dans du tertiobutylméthyléther on introduit, sous argon, sous agitation permanente, 0,5mole de bis-(2-méthoxy-éthoxy)-dihydroaluminate de sodium en solution à 70% dans du toluène. La solution est ensuite refroidie à la température de 0°C et on ajoute lentement 0,1 mole de 2-oximino-3-oxo-4-acétoxy-octadécanoate de méthyle. Le milieu réactionnel est alors chauffé progressivement jusqu'à 50°C. A la fin de la réaction, marquée par la disparition des produits de départ, le milieu réactionnel est hydrolysé à froid par une solution de soude. La phase aqueuse est décantée et la phase organique relavée par de l'eau, séchée, puis le solvant est évaporé sous vide. Après trituration dans de l'acétonitrile, on obtient un solide blanc qui, filtré et séché, donne 25g de 2-amino-octadécane-1,3,4-triol sous la forme d'une poudre blanche.
Rendement : 80%
Point de fusion: 132-134°C
Spectre RMN ¹³C (CDCl3):
   à = 14,21, CH3; δ = 23,09, CH3-CH2; δ = 26,02-26,39 (C12H27)-CH2-; δ = 29,8-30,14, C3H7-CH2)9-; δ = 32,4, C2H5-CH2- ; δ = 33,74-34,29, -CH2-CHOH ; δ = 53,11-55,8, CHNH2 ; δ = 63,97-65,3, CH2OH; δ = 71,97-75,88, CH(OH)-CH(OH).

### Exemple 2 : Synthèse du chlorhydrate du 2-amino-octadécane-1,3,4-triol

Dans du tétrahydrofurane on introduit, sous argon, sous agitation permanente, 0,275mole d'aluminohydrure de lithium. La solution est ensuite refroidie à la température de 0°C, et on ajoute lentement 0,055 mole de 2-oximino-3-oxo-4-acétoxy-octadécanoate de méthyle. Le milieu réactionnel est alors chauffé progressivement jusqu'à 60°C. A la fin de la réaction, marquée par la disparition des produits de départ, le milieu réactionnel est hydrolysé à froid par une solution d'acide chlorhydrique. Un précipité se forme, que l'on filtre, puis le solvant est évaporé sous vide. Après cristallisation dans un mélange d'éther éthylique et d'acétonitrile, on obtient un solide blanc qui, filtré et séché, donne 14,2g de chlorhydrate du 2-amino-octadécane-1,3,4-triol sous la forme d'une poudre blanche.
Rendement : 73%
Le produit se décompose au delà de 200°C
Spectre RMN ¹³C (CDCl3) :
   δ = 14,38, CH3 ; δ = 23,62, CH3-CH2 ; δ = 26,2-26,75 (C12H27)-CH2-; δ = 30,35-30,79, C3H7-(CH2)9- ; δ = 32,96, C2H5-CH2- ; δ = 34,37-35,32, -CH2-CHOH ; δ = 55,14-55,52, CHNH2; δ = 58,96-61,52, CH2OH; δ = 70,18-74,18, CH(OH)-CH(OH).

## Revendications

1. Procédé de préparation de 2-amino-alcane-1,3,4-triols ou des sels correspondants, caractérisée par le fait que l'on fait réagir en une seule étape un alkyl-2-oximino-3-oxo-4-acyloxyalcanoate de formule (I) : 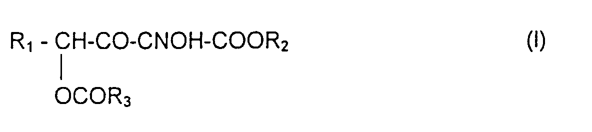 dans laquelle
R₁ représente un radical alcoyle, un radical alcényle ou un radical aralcoyle, éventuellement interrompu par des ponts éthers, linéaire ou ramifié, éventuellement hydroxylé et/ou porteur d'une fonction acyloxy en C₁ à C₈, comprenant 4 à 28 atomes de carbone,
R2 représente un radical alcoyle ou un radical alcényle, éventuellement interrompu par des ponts éthers, linéaire ou ramifié, comprenant 1 à 5 atomes de carbone,
R3 représente un radical alcoyle ou un radical alcényle, ou un radical aryle, linéaire ou ramifié, comprenant 1 à 6 atomes de carbone,
dans un solvant, en présence d'au moins un hydrure.

2. Procédé selon la revendication 1, dans lequel R₁ représente un radical alcoyle ou alcényle, éventuellement hydroxylé, ayant de 8 à 20 atomes de carbone.

3. Procédé selon l'une quelconque des revendications 1 et 2, dans lequel R₂ représente un radical méthyle ou éthyle.

4. Procédé selon l'une quelconque des revendications 1 à 3, dans lequel R₃ représente un radical méthyle ou phényle.

5. Procédé selon l'une quelconque des revendications 1 à 4, dans lequel la réaction s'effectue dans un solvant anhydre.

6. Procédé selon l'une quelconque des revendications 1 à 5, dans lequel le solvant est choisi parmi le toluène, l'heptane, le tetrahydrofurane, le tertiobutylméthyléther ou encore l'éther isopropylique.

7. Procédé selon la revendication 6, dans lequel le solvant est le tertiobutylméthyléther.

8. Procédé selon l'une quelconque des revendications 1 à 7, caractérisée par le fait que l'on travaille en atmosphère inerte.

9. Procédé selon la revendication 8, dans lequel l'atmosphère inerte est composée d'argon.

10. Procédé selon l'une quelconque des revendications 1 à 9, dans lequel l'hydrure est choisi parmi l'aluminohydrure de lithium et le bis-(2-méthoxy-éthoxy)-dihydroaluminate de sodium.

11. Procédé selon la revendication 10, dans lequel l'hydrure est du bis-(2-méthoxyéthoxy)-dihydroaluminate de sodium.

12. Procédé selon l'une quelconque des revendications 1 à 11, dans lequel l'hydrure est utilisé à une concentration allant de 3 à 7 équivalents-molaires par rapport à l'alkyl-2-oximino-3-oxo-4-acyloxyalcanoate.

13. Procédé selon l'une quelconque des revendications 1 à 12, dans lequel la réaction s'effectue à une température initiale contrôlée, puis se poursuit à toute température comprise entre -10°C et celle du reflux du solvant.

14. Procédé selon la revendication 13, dans lequel la réaction, une fois initiée, se poursuit à la température du reflux du solvant.

15. Procédé selon l'une quelconque des revendications 1 à 14, dans lequel la réaction s'initie à une température de 0° C.

16. Procédé selon l'une quelconque des revendications 1 à 15, dans lequel à la fin de la réaction le milieu réactionnel est amené à un pH inférieur à 2 ou supérieur à 11.

17. Procédé selon la revendication 16, dans lequel le pH du milieu réactionnel est voisin de 1.

18. Procédé selon la revendication 16, dans lequel le pH du milieu réactionnel est voisin de 12.

19. Procédé selon l'une quelconque des revendications 16 et 17 dans lequel on utilise de l'acide chlorhydrique.

20. Procédé selon l'une quelconque des revendications 16 et 18 dans lequel on utilise de la soude.

21. Procédé de préparation de céramides, caractérisé en ce que l'on met en oeuvre le procédé selon l'une quelconque des revendications précédentes, l'on acyle la fonction amine du 2-amino-alcane-1,3,4-triol ainsi obtenu par un chlorure d'acide, par un anhydride, par un ester de paranitrophénol, par un ester de succinimide, par un ester de dicyclohexylcarbodiimide, par un ester de méthyle ou d'éthyle ou par un azolide, en milieu anhydre ou dans un solvant tel que le tétrahydrofuranne, la pyridine, le diméthylformamide ou le dichlorométhane.

22. Procédé de préparation de céramides selon la revendication 21, caractérisé en ce que l'anhydride est un anhydride mixte et l'azolide est un imidazolide ou un pyrazolide.

## Claims

1. Process for the preparation of 2-amino-alkane-1,3,4-triols or of the corresponding salts, characterized in that an alkyl 2-hydroxyimino-3-oxo-4-acyloxyalkanoate of formula (I): 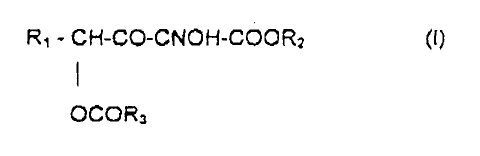 in which
R₁ represents an alkyl radical, an alkenyl radical or an aralkyl radical which is optionally interrupted by ether bridges, which is linear or branched, which is optionally hydroxylated and/or a carrier of a C₁ to C₈ acyloxy functional group and which comprises 4 to 28 carbon atoms,
R₂ represents an alkyl radical or an alkenyl radical which is optionally interrupted by ether bridges, which is linear or branched and which comprises 1 to 5 carbon atoms,
R₃ represents an alkyl radical or an alkenyl radical or an aryl radical which is linear or branched and which comprises 1 to 6 carbon atoms,
is reacted, in a single stage, in a solvent, in the presence of at least one hydride.

2. Process according to Claim 1, in which R₁ represents an optionally hydroxylated alkyl or alkenyl radical having from 8 to 20 carbon atoms.

3. Process according to either one of Claims 1 and 2, in which R₂ represents a methyl or ethyl radical.

4. Process according to any one of Claims 1 to 3, in which R₃ represents a methyl or phenyl radical.

5. Process according to any one of Claims 1 to 4, in which the reaction is carried out in an anhydrous solvent.

6. Process according to any one of Claims 1 to 5, in which the solvent is chosen from toluene, heptane, tetrahydrofuran, tert-butyl methyl ether or alternatively isopropyl ether.

7. Process according to Claim 6, in which the solvent is tert-butyl methyl ether.

8. Process according to any one of Claims 1 to 7, characterized in that the reaction is carried out in an inert atmosphere.

9. Process according to Claim 8, in which the inert atmosphere is composed of argon.

10. Process according to any one of Claims 1 to 9, in which the hydride is chosen from lithium aluminium hydride and sodium bis(2-methoxyethoxy)aluminium hydride.

11. Process according to Claim 10, in which the hydride is sodium bis(2-methoxyethoxy)aluminium hydride.

12. Process according to any one of Claim 1 to 11, in which the hydride is used at a concentration ranging from 3 to 7 molar equivalents with respect to the alkyl 2-hydroxyimino-3-oxo-4-acyloxyalkanoate.

13. Process according to any one of Claims 1 to 12, in which the reaction is carried out at a controlled starting temperature and is then continued at any temperature between -10°C and that of reflux of the solvent.

14. Process according to Claim 13, in which the reaction, once initiated, is continued at the reflex temperature of the solvent.

15. Process according to any one of Claims 1 to 14, in which the reaction is initiated at a temperature of 0°C.

16. Process according to any one of Claims 1 to 15, in which, at the end of the reaction, the reaction mixture is brought to a pH of less than 2 or greater than 11.

17. Process according to Claim 16, in which the pH of the reaction mixture is in the region of 1.

18. Process according to Claim 16, in which the pH of the reaction mixture is in the region of 12.

19. Process according to either one of Claims 16 and 17, in which hydrochloric acid is used.

20. Process according to either one of Claims 16 and 18, in which sodium hydroxide is used.

21. Process for the preparation of ceramides, characterized in that the process according to any one of the preceding claims is used and the amine functional group of the 2-aminoalkane-1,3,4-triol thus obtained is acylated by an acid chloride, by an anhydride, by a paranitrophenol ester, by a succinimide ester, by a dicyclohexylcarbodiimide ester, by a methyl or ethyl ester or by an azolide, in anhydrous medium or in a solvent, such as tetrahydrofuran, pyridine, dimethylformamide or dichloromethane.

22. Process for the preparation of ceramides according to Claim 21, characterized in that the anhydride is a mixed anhydride and the azolide is an imidazolide or a pyrazolide.

## Patentansprüche

1. Verfahren zur Herstellung von 2-Amino-1,3,4-alkantriolen oder deren Salzen, dadurch gekennzeichnet, daß ein Alkyl-2-oximino-3-oxo-4-acyloxyalkanoat der Formel (I): 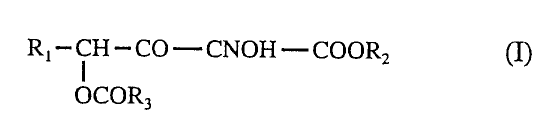 in der
R₁ eine lineare oder verzweigte und gegebenenfalls durch Etherbrücken unterbrochene Alkyl-, Alkenyl- oder Arylalkylgruppe mit 4 bis 28 Kohlenstoffatomen darstellt, die gegebenenfalls hydroxyliert sein und/oder in C₁- bis C₆-Stellung eine Acyloxyfunktion tragen kann,
R₂ eine lineare oder verzweigte und gegebenenfalls durch Etherbrücken unterbrochene Alkyl- oder Alkenylgruppe mit 1 bis 5 Kohlenstoffatomen darstellt,
und R₃ eine lineare oder verzweigte Alkyl-, Alkenyl- oder Arylgruppe mit 1 bis 6 Kohlenstoffatomen darstellt,
in einem Lösungsmittel in Gegenwart mindestens eines Hydrids in einem einzigen Reaktionsschritt umgesetzt wird.

2. Verfahren nach Anspruch 1, bei dem R₁ eine gegebenenfalls hydroxylierte Alkyl- oder Alkenylgruppe mit 8 bis 20 Kohlenstoffatomen darstellt.

3. Verfahren nach einem der Ansprüche 1 und 2, bei dem R₂ eine Methyl- oder Ethylgruppe darstellt.

4. Verfahren nach einem der Ansprüche 1 bis 3, bei dem R₃ eine Methyl- oder Phenylgruppe darstellt.

5. Verfahren nach einem der Ansprüche 1 bis 4, bei dem die Reaktion in einem wasserfreien Lösungsmittel stattfindet.

6. Verfahren nach einem der Ansprüche 1 bis 5, bei dem das Lösungsmittel ausgewählt wird aus Toluol, Heptan, Tetrahydrofuran, tert.-Butyl-methylether sowie Diisopropylether.

7. Verfahren nach Anspruch 6, bei dem als Lösungsmittel tert.-Butyl-methylether verwendet wird.

8. Verfahren nach einem der Ansprüche 1 bis 7, gekennzeichnet durch die Tatsache, daß unter Schutzgas gearbeitet wird.

9. Verfahren nach Anspruch 8, bei dem als Schutzgas Argon verwendet wird.

10. Verfahren nach einem der Ansprüche 1 bis 9, bei dem das Hydrid ausgewählt wird aus Lithiumaluminiumhydrid und Natrium-bis-(2-methoxyethoxy)-dihydroaluminat.

11. Verfahren nach Anspruch 10, bei dem als Hydrid Natrium-bis-(2-methoxyethoxy)-dihydroaluminat verwendet wird.

12. Verfahren nach einem der Ansprüche 1 bis 11, bei dem das Hydrid in einer Konzentration von 3 bis 7 mol pro Mol Alkyl-2-oximino-3-oxo-4-acyloxyalkanoat eingesetzt wird.

13. Verfahren nach einem der Ansprüche 1 bis 12, bei dem die Reaktion bei einer kontrollierten Anfangstemperatur initiiert und anschließend bei einer beliebigen Temperatur zwischen - 10°C und der Rückflußtemperatur des Lösungsmittels fortgeführt wird.

14. Verfahren nach Anspruch 13, bei dem die Reaktion, sobald sie initiiert wurde, bei der Rückflußtemperatur des Lösungsmittels fortgeführt wird.

15. Verfahren nach einem der Ansprüche 1 bis 14, bei dem die Reaktion bei 0°C initiiert wird.

16. Verfahren nach einem der Ansprüche 1 bis 15, bei dem der pH-Wert des Reaktionsmediums nach Beendigung der Reaktion auf kleiner 2 oder größer 11 eingestellt wird.

17. Verfahren nach Anspruch 16, bei dem der pH-Wert des Reaktionsmediums ungefähr 1 beträgt.

18. Verfahren nach Anspruch 16, bei dem der pH-Wert des Reaktionsmediums ungefähr 12 beträgt.

19. Verfahren nach einem der Ansprüche 16 und 17, bei dem Salzsäure verwendet wird.

20. Verfahren nach einem der Ansprüche 16 und 18, bei dem Natronlauge verwendet wird.

21. Verfahren zur Herstellung von Ceramiden, dadurch gekennzeichnet, daß das Verfahren nach einem der vorhergehenden Ansprüche ausgeführt wird und daß die Aminogruppe des so erhaltenen 2-Amino-1,3,4-alkantriols mit Hilfe eines Säurechlorids, eines Anhydrids, eines p-Nitrophenolesters, eines Succinimidesters, eines Dicyclohexylcarbodiimidesters, eines Methyl- oder Ethylesters oder eines Azolids in wasserfreiem Medium oder in einem Lösungsmittel wie Tetrahydrofuran, Pyridin, Dimethylformamid oder Dichlormethan acyliert wird.

22. Verfahren zur Herstellung von Ceramiden nach Anspruch 21, dadurch gekennzeichnet, daß das Anhydrid ein gemischtes Anhydrid und das Azolid ein Imidazolid oder ein Pyrazolid ist.
